# EUROPEAN PATENT APPLICATION

(11) **EP 4 625 419 A1**
(43) Date of publication of application: **01.10.2025**
(21) Application number: 23894000.1
(22) Date of filing: 24.11.2023
(51) Int. Cl.: G16B 25/10, G16B 35/10, G16B 35/20, G16B 40/20, G16B 20/00, A61K 36/00, C12N 1/00, C12N 15/00, G06N 3/12

(54) **CODON OPTIMIZATION**

(30) Priority: 24.11.2022 CN 202211485518
(71) Applicant: Nanjing GenScript Biotech Co., Ltd., Nanjing, Jiangsu 211100 (CN)
(72) Inventor: FAN, Long, Nanjing, Jiangsu 211100 (CN); HE, Yuzhuo, Nanjing, Jiangsu 211100 (CN); ZHANG, Lihua, Nanjing, Jiangsu 211100 (CN); LI, Hong, Nanjing, Jiangsu 211100 (CN); TAN, Hao, Nanjing, Jiangsu 211100 (CN); CHEN, Zhiwei, Nanjing, Jiangsu 211100 (CN)
(74) Representative: Synergy IP Group AG
(86) International application number: PCT/CN2023/133868
(87) International publication number: WO 2024/109911

(57) **Abstract**

Provided is a technique relating to codon optimization. A method for optimizing a nucleic acid sequence for expression of a protein in a host comprises: obtaining a protein subsequence-nucleic acid subsequence pair according to collected highly expressed protein sequences and encoding nucleic acid sequences thereof, so as to form a training set; using the training set to train a neural machine translation model, wherein the neural machine translation model is used to realize translation from an amino acid sequence to a codon sequence; cleaving the protein sequence requiring codon optimization into protein subsequences; using the trained neural machine translation model to translate the protein subsequences from amino acid sequences to codon sequences; and overlapping the translated subsequences, so as to combine same into a full-length codon sequence, wherein during overlapping to synthesize the codon sequence, the synonymous codon with the highest occurrence frequency or number is selected as the optimal codon for the position according to the frequency or the number of the synonymous codon corresponding to each amino acid position of the protein sequence.

## Description

### Cross-Reference to Related Applications

The present invention claims priority to Chinese Patent Application No. 202211485518.X filed on November 24, 2022, the entire content of which is incorporated herein by reference.

### Technical Field

The present disclosure relates to protein expression in biotechnology, and more particularly relates to a method for optimizing a nucleic acid sequence for expression of a protein in a host.

### Background Art

### 1. Current status of codon optimization algorithm

Codon optimization is a technique that improves the use effect of a nucleic acid by selecting suitable synonymous codons that are suitable for a specific expression host system without changing an amino acid encoding sequence, which is very important for increasing the expression level of recombinant proteins and antibodies and the use effect of DNA vaccines. These applications and their effects are described in: Mauro, V. P. and Chappell, S. A. (2014) A critical analysis of codon optimization in human therapeutics. Trends Mol. Med., 20, 604-613 and Angov, E. (2011) Codon usage: nature's roadmap to expression and folding of proteins. Biotechnol. J., 6, 650-659, the contents of which are hereby incorporated by reference into the present disclosure.

With the emergence of mRNA-based therapies and vaccines, codon optimization can also be used to improve the stability and expression level of mRNA, and reduce the immunogenicity of mRNA. The relevant applications are described in: To, K. K. and Cho, W. C. (2021) An overview of rational design of mRNA-based therapeutics and vaccines. Expert Opin. Drug Discov., 16, 1307-1317 and Weissman, D. (2015) mRNA transcript therapy. Expert Rev. Vaccines, 14, 265-281, the contents of which are hereby incorporated by reference into the present disclosure.

Codon optimization algorithms are divided into two categories depending on whether they are global optimizations: The first category is to optimize a complete protein sequence as a whole, which is called global optimization, such as GenSmart codon optimization tool from GenScript (see WO 2020/024917 A1, the content of which is hereby incorporated by reference into the present disclosure), JCat (see Grote, A., Hiller, K., Scheer, M., Münch, R., Nörtemann, B., Hempel, D.C. and Jahn, D. (2005) JCat: a novel tool to adapt codon usage of a target gene to its potential expression host. Nucleic Acids Res., 33, W526-531, the content of which is hereby incorporated by reference into the present disclosure) and CHARMING (see Wright, G., Rodriguez, A., Li, J., Milenkovic, T., Emrich, S. J. and Clark, P. L. (2022) CHARMING: Harmonizing synonymous codon usage to replicate a desired codon usage pattern. Protein Sci., 31, 221-231, the content of which is hereby incorporated by reference into the present disclosure); the second category is to use local fragments of a protein sequence as optimization units, that is, after a complete protein sequence is divided into subsequences by means of sequence slicing or a sliding window, each subsequence is first optimized, and the optimized subsequences are then combined and spliced into a complete sequence, which is called local optimization, such as GeneOptimizer from GeneArt (see Raab, D., Graf, M., Notka, F., Schödl, T. and Wagner, R. (2010) The GeneOptimizer Algorithm: using a sliding window approach to cope with the vast sequence space in multiparameter DNA sequence optimization. Syst. Synth. Biol., 4, 215-225, the content of which is hereby incorporated by reference into the present disclosure) and codon optimization method based on immune algorithm (see WO 2019/020054 A1, the content of which is hereby incorporated herein by reference into the present disclosure).

Codon optimization algorithms can also be divided into two categories depending on whether they use an end-to-end (or sequence-to-sequence) model framework. The first category is a non-end-to-end optimization method, which optimizes one or more manually extracted feature parameters of a sequence by combinatorial optimization methods such as heuristic algorithms (e.g., genetic algorithm, particle swarm optimization, simulated annealing) or exhaustive methods. In the optimization process, the feature parameters of the sequence are used to correlate traits such as protein expression level, mRNA stability, and thereby evaluate the advantages and disadvantages of the optimization output results. Common feature parameters include CAI (codon adaptation index), codon context, CBI (codon bias index), ENC (effective number of codon), FOP (frequency of optimal codons), CPP (codon preference parameter), tAI (tRNA adaptation index), the number of hidden stop codon, GC content (content of G and C bases), rare codon content, the number of mRNA inhibitory regulatory motifs, mRNA secondary structure (mainly including hairpin structure and folding free energy), key codons and mathematical modeling and scoring in machine learning, microRNA binding sites, G4 content (G-quadruplex content), etc. (see Gould, N., Hendy, O. and Papamichail, D. (2014) Computational tools and algorithms for designing customized synthetic genes. Front. Bioeng. Biotechnol., 2, 41, the content of which is hereby incorporated by reference into the present disclosure). Such software include the GenScript GenSmart codon optimization tool (see WO 2020/024917 A1), COOL (see Chin, J. X., Chung, B. K.-S. and Lee, D.-Y. (2014) Codon Optimization OnLine (COOL): a web-based multi-objective optimization platform for synthetic gene design. Bioinformatics, 30, 2210-2212, the content of which is hereby incorporated by reference into the present disclosure) and GeneArt GeneOptimizer (see Raab, D., Graf, M., Notka, F., Schödl, T. and Wagner, R. (2010) The GeneOptimizer Algorithm: using a sliding window approach to cope with the vast sequence space in multiparameter DNA sequence optimization. Syst. Synth. Biol., 4, 215-225). The second category is an end-to-end optimization method, which, inspired by neural machine translation, does not require the manual extraction of sequence features, but borrows the encoder-decoder algorithm framework of natural language processing to realize direct correspondence transformation from protein sequences to nucleic acid sequences, that is, the input protein sequence is directly output as the corresponding nucleic acid sequence by algorithms. The relevant optimization tools include RNN-based codon optimization (see Goulet, D. R., Yan, Y., Agrawal, P., Waight, A. B., Mak, A. N. and Zhu, Y. (2022) Codon Optimization Using a Recurrent Neural Network. J. Comput. Biol. and Jain, R., Jain, A., Mauro, E., LeShane, K. and Densmore, D. (2021) ICOR: Improving codon optimization with recurrent neural networks. bioRxiv*,* the contents of which are hereby incorporated by reference into the present disclosure), BiLSTM-based codon optimization (see Fu, H., Liang, Y., Zhong, X., Pan, Z., Huang, L., Zhang, H., Xu, Y., Zhou, W. and Liu, Z. (2020) Codon optimization with deep learning to enhance protein expression. Sci. Rep., 10, 1-9, the content of which is hereby incorporated by reference into the present disclosure) and ANN-based codon optimization (see Tarakaram, Y., Mounika, Y., Prasanna, Y. L. and Singh, T. (2021) Codon Optimization and Converting DNA Sequence into Protein Sequence using Deep Neural Networks. In. IEEE, pp. 1-5, the content of which is hereby incorporated by reference into the present disclosure).

### 2. Neural machine translation

Neural Machine Translation (NMT) is a machine translation that introduces artificial neural networks for translation, whose performance significantly exceeds statistical machine translation in many translation applications. The prototype of this method was conceived and proposed in the late 1980s by the IBM T.J. Watson Research Center and was implemented in the first academic paper in 2013. Currently, neural machine translation models generally consist of an encoder-decoder framework. Compared with statistical machine translation, the neural machine translation model is simple in composition, does not require feature engineering and translation rule extraction, and solves problems of long-range memory and complex character alignment between two texts; moreover, the model occupies less memory and supports incremental training (see Mohamed, S. A., Elsayed, A. A., Hassan, Y. and Abdou, M. A. (2021) Neural machine translation: past, present, and future. Neural Comput. Appl., 33, 15919-15931, the content of which is hereby incorporated by reference into the present disclosure). The encoder-decoder framework of the neural machine translation model can generally consist of CNN, RNN, GRU, LSTM and BiLSTM. It can also incorporate self-attention or pre-trained models such as BERT, RoBERTa, XLNet or GPT2, or adopt the transformer framework. Toolkits commonly used for building neural machine translation include OpenNMT, Nmt, XNMT, Nematus, SOCKEYE, Marian, Fairseq, Neural monkey, Tensor2tensot, NMT-KERAS, THUMT, etc. (see Tan, Z., Wang, S., Yang, Z., Chen, G., Huang, X., Sun, M. and Liu, Y. (2020) Neural machine translation: A review of methods, resources, and tools. AI Open, 1, 5-21, Singh, S. P., Kumar, A., Darbari, H., Singh, L., Rastogi, A. and Jain, S. (2017) Machine translation using deep learning: An overview. In. IEEE, pp. 162-167, and Zhu, J., Xia, Y., Wu, L., He, D., Qin, T., Zhou, W., Li, H. and Liu, T.-Y. (2020) Incorporating bert into neural machine translation. ArXiv Prepr. ArXiv200206823*,* the contents of which are hereby incorporated by reference into the present disclosure).

### 3. Ribosome profiling

Ribosome profiling/Ribo-seq technology, invented in 2009, is a technique for studying the binding of ribosomes to mRNA by using deep sequencing techniques, and can be used to detect and globally monitor protein translation *in vivo,* such as ribosome stalling site discovery, ORF verification, translation extension and termination mechanism studies (see Brar, G. A. and Weissman, J. S. (2015) Ribosome profiling reveals the what, when, where and how of protein synthesis. Nat. Rev. Mol. Cell Biol., 16, 651-664, the content of which is hereby incorporated by reference into the present disclosure). During library construction of Ribo-seq sequencing samples, the ribosome-binding fragments (protected mRNA fragments) generated generally show a main peak of about 30 nt during deep sequencing. There are slight differences among different species. For example, the Ribo-seq fragments from *S. cerevisiae* show a main peak of 28-29 nt, while the Ribo-seq fragments from mammalian cells show a main peak of 30-31 nt. Additionally, for some species, the Ribo-seq fragments show a main peak between 15 nt and 65 nt. (see Jackson, R. and Standart, N. (2015) The awesome power of ribosome profiling. Rna, 21, 652-654, the content of which is hereby incorporated by reference into the present disclosure). The finding shows that the lengths of mRNA fragments directly occupied (bound) by small and large ribosomal subunits or intact ribosomes is mainly concentrated between 15 nt and 66 nt.

The basic logic of using the neural machine translation model to solve the codon optimization problem is to treat the protein sequence as a natural language and the encoded nucleic acid sequence as another natural language, and then complete the end-to-end translational transformation process through the neural machine translation model. However, there are currently the following problems:
(1) compared with the translation of traditional natural languages, the complexity of the constituent units of protein sequences and DNA sequences is lower (that is, there are only 20 common amino acids and only 4 nucleotides) and the sequences are contiguous; there is a one-to-many mapping relationship between amino acids and synonymous codons; there are no complex grammatical rules for limitation.
(2) For a single protein expression host, there are few corresponding training sets (or corpora), especially for lower organisms. For example, even if all the protein-coding genes of *E.coli* K12 substr. MG1655 are used, there are only more than 4300 proteins, which is insufficient as a training set (or corpus) for a deep network.
(3) There is a large difference in the lengths of protein sequences. For example, the smallest protein in *E. coli* is the K+ transport (KdpF) constituent protein, which contains only 29 amino acids, while the largest protein is the RNase T protein, which contains 1538 amino acids. Currently, the neural machine translation methods applied to codon optimization all directly use the whole protein sequence as an input for global optimization, while neural machine translation models for global optimization are at risk of gradually decreasing the effect of long-range memory as the length of the protein sequence increases.
(4) There is no integration of existing biological research findings, and there is no explicit biological logical support.

As a result of the accumulation of the problems above, the existing codon optimization methods based on neural machine translation models have not achieved an improved use effect, and for details, reference is made to the evaluation results of RNN-based codon optimization methods and the widely-used centralized codon optimization tools in the industry - GenSmart codon optimization tool from GenScript and GeneOptimizer from GeneArt (see Goulet, D. R., Yan, Y., Agrawal, P., Waight, A. B., Mak, A. N. and Zhu, Y. (2022) Codon Optimization Using a Recurrent Neural Network. J. Comput. Biol.*).*

Therefore, there is a need for a codon optimization technique that can take advantage of the development achievements of codon optimization algorithms to overcome the various defects noted above.

### Summary of the Invention

To improve the use effect of neural machine translation in codon optimization, the present invention proposes a codon optimization method for local optimization based on a neural machine translation model and a sliding window. In the method, the size of the sliding window is set according to the size of the main peak of ribosome binding fragments (protected mRNA fragments) in ribosome profiling/Ribo-seq, the sliding window slides along a protein sequence to generate sub-fragments, then a transformer model containing self-attention and positional encoding (see Lin, T., Wang, Y., Liu, X. and Qiu, X. (2021) A survey of transformers. ArXiv Prepr. ArXiv210604554 and Tay, Y., Dehghani, M., Bahri, D. and Metzler, D. (2020) Efficient transformers: A survey. ACM Comput. Surv. CSUR*,* the contents of which are hereby incorporated by reference into the present disclosure) is used to perform sequence-to-sequence translation on the sub-fragments of the protein sequence, the translated sub-fragment sequences are then overlapped, the synonymous codon with a high frequency at the amino acid position is preferentially selected and used according to the frequency of the synonymous codon corresponding to each amino acid position, and finally, the subsequences are combined into a full-length sequence.

According to a first aspect of the present disclosure, a computer-implemented method for optimizing a nucleic acid sequence for expression of a protein in a host is provided. The method may comprise: obtaining a protein subsequence-nucleic acid subsequence pair according to collected highly expressed protein sequences and encoding nucleic acid sequences thereof, so as to form a training set; using the training set to train a neural machine translation model, wherein the neural machine translation model is used to realize translation from an amino acid sequence to a codon sequence; cleaving the protein sequence requiring codon optimization into protein subsequences; using the trained neural machine translation model to translate the protein subsequences from amino acid sequences to codon sequences; and overlapping the translated subsequences, so as to combine same into a full-length codon sequence. The synonymous codon with the highest occurrence frequency or number is selected as the optimal codon for the position according to the frequency or the number of the synonymous codon corresponding to each amino acid position of the protein sequence.

In the method according to the first aspect of the present disclosure, the step of forming a training set may further comprise: separately adding padding amino acid sequences at both ends of the protein sequence; separately adding corresponding padding codons at both ends of the encoding nucleic acid sequence; using a sliding window to perform subsequence extraction on the protein sequences and the encoding nucleic acid sequences thereof; tokenizing the protein subsequences with amino acids as the minimum unit; tokenizing the nucleic acid subsequences with codons as the minimum unit; adding a sentence start flag and a sentence end flag before and after each of the protein subsequences and the nucleic acid subsequences, respectively, to form a training set.

In the above step, preferably, the padding amino acid sequence consists of N amino acids; the length of the sliding window on the protein sequence is L1 amino acids and L1 = N + 1, with a sliding step size of 1 amino acid; the length of the sliding window on the encoding nucleic acid sequence is L2 nucleotides and L2 = 3 × L1, with a sliding step size of 3 nucleotides.

Preferably, the padding amino acid sequence may consist of N consecutive methionine or N consecutive tryptophan.

When the padding amino acid is methionine, the corresponding padding codon is ATG. When the padding amino acid is tryptophan, the corresponding padding codon is TGG.

Preferably, the length L1 of the sliding window on the protein sequence is 5 to 22 amino acids and the length L2 of the sliding window on the encoding nucleic acid sequence is 15 to 66 nucleotides.

In the method according to the first aspect of the present disclosure, the neural machine translation model may be a transformer model.

In addition, the neural machine translation model may be an artificial neural network machine translation model containing self-attention and positional encoding.

In the method according to the first aspect of the present disclosure, the step of cleaving the protein sequence requiring codon optimization into protein subsequences further comprises: separately adding padding amino acid sequences at both ends of the protein sequence requiring codon optimization; using a sliding window to cleave the protein sequence into protein subsequences.

In the above step, preferably, the padding amino acid sequence consists of N amino acids; the length of the sliding window is L1 amino acids and L1 = N + 1. By adding the padding amino acid sequence, each amino acid of the protein sequence is scanned the same number of times by the sliding window, and each amino acid will be included in the L1 cleaved protein subsequences. In the step of translating the protein subsequences from amino acid sequences to codon sequences, the amino acid at each position of the protein sequence is translated L1 times by the trained neural machine translation model.

Preferably, the padding amino acid sequence consists of N consecutive methionine or N consecutive tryptophan.

Similar to the step of forming the training set, in the step of cleaving the protein sequence requiring codon optimization into subsequences, preferably, the length L1 of the sliding window is 5 to 22 amino acids.

In the method according to the first aspect of the present disclosure, in the step of overlapping the translated subsequences so as to combine same into a full-length codon sequence, the synonymous codon with the highest occurrence frequency or number after L1 translations of each amino acid in the L1 subsequences of the protein sequence is selected as the optimal codon for the amino acid position, and thus the sequence consisting of the optimal codons for all amino acids is used as the synthesized full-length codon sequence.

In the method according to the first aspect of the present disclosure, for one amino acid position, when two or more synonymous codons occur at the same frequency or number, the optimal codon for the position is selected according to at least one of the following criteria: codon preference, GC%, and folding energy ordering of nucleic acid sub-fragments.

The method according to the first aspect of the disclosure may further comprise: performing synonymous codon substitution for deleterious feature sequences, enzyme cleavage sites for molecular cloning operations, or regions capable of producing alternative splicing sites in expression hosts.

According to a second aspect of the present disclosure, a non-transitory computer-readable storage medium for storing a computer program is provided. The computer program comprises instructions that, when executed by a processor of an electronic device, cause the electronic device to implement the method according to the first aspect of the present disclosure.

According to a third aspect of the present disclosure, a system for optimizing a nucleic acid sequence for expression of a protein in a host is provided. The system comprises: a processor; a memory; and a computer program. The computer program is stored in the memory and configured to be executed by the processor, and the computer program comprises instructions for implementing the method according to the first aspect of the present disclosure.

According to a fourth aspect of the present disclosure, an electronic device for optimizing a nucleic acid sequence for expression of a protein in a host is provided, wherein the device comprises a tool for implementing the method according to the first aspect of the present disclosure.

According to a fifth aspect of the present disclosure, a computer program product stored on a recordable medium for optimizing a nucleic acid sequence for expression of a protein in a host is provided, wherein the computer program product comprises computer software for implementing the method according to the first aspect of the present disclosure.

According to a sixth aspect of the present disclosure, an isolated nucleic acid molecule comprising the optimized nucleic acid sequence obtained by the method according to the first aspect of the present disclosure is provided.

According to a seventh aspect of the present disclosure, a vector comprising the isolated nucleic acid molecule according to the sixth aspect of the present disclosure is provided.

According to an eighth aspect of the present disclosure, a recombinant host cell comprising the isolated nucleic acid molecule according to the sixth aspect of the present disclosure or the vector according to the seventh aspect of the present disclosure is provided.

According to a ninth aspect of the present disclosure, a method for expressing a protein in a host cell is provided. The method comprises: using the method according to the first aspect of the present disclosure to obtain an optimized nucleic acid sequence for expression of the protein in the host cell; synthesizing a nucleic acid molecule comprising the optimized nucleic acid sequence; introducing the nucleic acid molecule into the host cell to obtain a recombinant host cell; and culturing the recombinant host cell under conditions that allow expression of the protein from the optimized nucleic acid sequence.

According to the codon optimization technique of the present disclosure, the complexity of neural machine translation is reduced and the effectiveness is improved by continuous local optimization. Self-attention and positional encoding are used in a neural machine translation model such as transformer or variants thereof, such that the translation effect can be improved. During local optimization, the size of the sliding window is determined based on the conclusions of Ribo-Seq, the interaction between ribosomes and mRNA in the biological process of protein synthesis is simulated, and subsequences are generated using the sliding window. Finally, the optimal codon is selected according to the occurrence number/frequency of synonymous codons at a certain position, which essentially integrates the process of local folding and local unfolding of mRNA, and features such as the codon usage bias and dicodon usage bias involved in this process.

### Brief Description of the Drawings

The present invention will be more fully understood from the following detailed description in combination with the accompanying drawings, in which similar elements are numbered in a similar manner, where:
FIG. 1 is a flowchart of a method for optimizing a nucleic acid sequence for expression of a protein in a host according to the example of the present invention.
FIG. 2 is a schematic diagram of an embodiment used during the implementation of the method of FIG. 1, in which when the sliding window is 3 amino acids, 2 M are added at both ends of the sequence.
FIG. 3 is a schematic diagram of an embodiment used during the implementation of the method of FIG. 1, in which when the sliding window is 3 amino acids, the sliding step size is 1, the sequence is read sequentially, and each amino acid is read 3 times.
FIG. 4 is a schematic diagram of the operation of the transformer framework.
FIG. 5 is a graph showing the results of the expression levels of M2-1 proteins obtained by different codon optimization methods.

### Detailed Description of Embodiments

Unless otherwise stated, the technical and scientific terms used in the present disclosure have the same meaning as commonly understood by one of ordinary skill in the art to which the present invention pertains.

The technical solutions of the present invention are further illustrated in more detail by the examples and in conjunction with the accompanying drawings. Unless otherwise stated, the methods and materials in the examples described below are conventional products that can be purchased from the market. Those skilled in the art of the present invention would understand that the methods and materials described below are exemplary only and should not be considered as limiting the scope of the present invention.

The present invention provides a codon optimization method for local optimization based on a neural machine translation model and a sliding window.

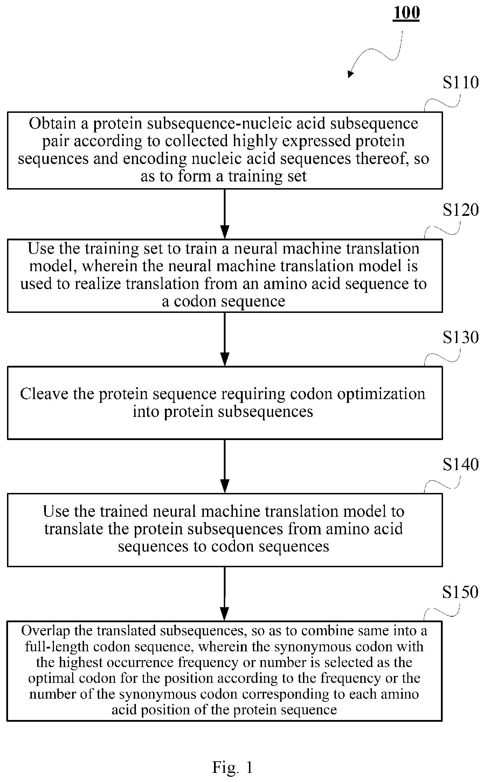

FIG. 1 is a flowchart of a method for optimizing a nucleic acid sequence for expression of a protein in a host according to the example of the present invention.

As shown in FIG. 1, method 100 for optimizing a nucleic acid sequence for expression of a protein in a host according to the example of the present invention begins with step S110. In step S110, a protein subsequence-nucleic acid subsequence pair is obtained according to collected highly expressed protein sequences and encoding nucleic acid sequences thereof, so as to form a training set. This step corresponds to the stage of preparation of a training set (i.e., corpus) for implementing the method of the present invention, which will be described in detail below.

### Stage 1. Preparation of training set (i.e., corpus)

First, highly expressed protein sequences and encoding nucleic acid sequences thereof are collected. For example, highly expressed protein sequences and encoding nucleic acid sequences thereof can be collected according to the analysis results and annotation information of the proteome, transcriptome and genome of a protein expression host. For each of the highly expressed protein sequences and encoding nucleic acid sequences thereof, the following processing is performed.
(1) Padding amino acid sequences are separately added at both ends of the protein sequence, while corresponding padding codons are separately added at both ends of the encoding nucleic acid sequence.

Specifically, N padding amino acids may be added at both ends of the protein sequence. The padding amino acids can consist of N amino acids randomly, generally N consecutive M (methionine/met) or W (tryptophan). Here, those skilled in the art will recognize that the purpose of padding amino acids at the beginning and end of a protein sequence is to enable the first and last amino acids in the sequence to also be read multiple times (the same number of times as the other amino acids in the sequence). The reason for padding M or W is that they correspond to the start codon and can promote high expression. Correspondingly, corresponding padding codons are added at both ends of the encoding nucleic acid sequence, i.e., ATG for M and TGG for W.

(2) A sliding window is used to perform subsequence extraction on the protein sequences and the encoding nucleic acid sequences thereof.

A sliding window is used to perform subsequence extraction on the protein sequences. The length of the sliding window on the protein sequence is L1, generally 5-22 amino acids. Here, it should be noted that under natural conditions, the length of the ribosome sliding window is 5, and a training set can occur when the length of the sliding window is greater than 2, preferably greater than 7. The number of 5-22 selected for the sliding window here is based on the number of amino acids corresponding to the main length of the ribosome-binding fragments in Ribo-seq of different species. The relationship between the length of the sliding window and the number of padding amino acids is L1 = N + 1. The sliding step size for the subsequence extraction on the protein sequences is 1 amino acid. At the same time, a sliding window is used to perform subsequence extraction on the encoding nucleic acid sequences. The length of the sliding window on the encoding nucleic acid sequence is L2 nucleotides, generally 15-66 nucleotides, which is three times the size L1 of the sliding window on the protein sequence, i.e., L2 = 3 × L1. The sliding step size for the subsequence extraction on the encoding nucleic acid sequences is 3 nucleotides.

More generally, the sliding window size (i.e., length) is consistent with the main length of the ribosome-binding fragments in ribosome profiling (Ribo-seq), and is also the size of the main region where the ribosome interacts with mRNA during protein biosynthesis; correspondingly, the sliding step size is consistent with a protein biosynthesis process in which the ribosome slides along the mRNA by the length of a single codon during protein translation. Here, the sliding of the sliding window is actually used to mimic the process in which the ribosome slides along the mRNA during protein translation, and therefore selecting a sliding window having a size similar to that of ribosome occupancy is more biomimetic. Thus, the AI algorithm of the present invention also achieves better performance than other AI algorithms.

(3) Protein subsequences are tokenized with amino acids as the minimum unit, and nucleic acid subsequences are tokenized with codons as the minimum unit.

(4) A sentence start flag and a sentence end flag are added before and after each of the protein subsequences and the nucleic acid subsequences, respectively, to form a training set (i.e., corpus).

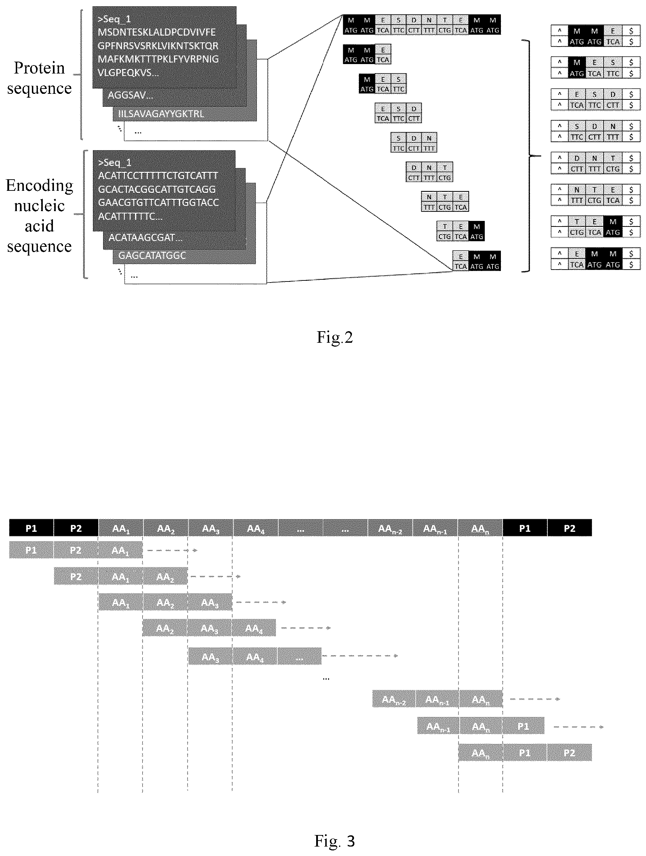

FIG. 2 illustrates the operation of the above stage. Specifically, FIG. 2 is a schematic diagram of an embodiment used during the implementation of the method of FIG. 1, in which when the length of the sliding window on the protein sequences is 3 amino acids, 2 M are added at both ends of the sequence. Assuming that a certain protein expression host has a highly expressed protein having a protein sequence of ESDNTE and an encoding nucleic acid sequence of TCATTCCTTTTTCTGTCA, when N = 2, the padding amino acid is M, the length L1 of the sliding window on the protein sequence = 3, the length L2 of the sliding window on the encoding nucleic acid sequence = 9, the sentence start flag is "^", and the sentence end flag is "$", after sequence padding, sliding window scanning, tokenizing and special flag addition, 8 protein subsequence-nucleic acid subsequence pairs are obtained and added to the training set (corpus).

Next, still referring to FIG. 1, the method proceeds to step S120. In step S120, the training set is used to train a neural machine translation model. The neural machine translation model is used to realize translation from an amino acid sequence to a codon sequence. Specifically, see the detailed description of model training and end-to-end translation below.

### Stage 2. Model training and end-to-end translation

According to a preferred embodiment of the present invention, a transformer model may be selected for the neural machine translation model during model training and subsequent model application, i.e., translation of protein sequences. The transformer model (see Vaswani, A., Shazeer, N., Parmar, N., Uszkoreit, J., Jones, L., Gomez, A. N., Kaiser, L. and Polosukhin, I. (2017) Attention is all you need. Adv. Neural Inf. Process. Syst., 30**,** the content of which is hereby incorporated by reference into the present disclosure) and variants thereof are widely applied in the fields such as computer vision, video, text and speech (Xu, Y., Wei, H., Lin, M., Deng, Y., Sheng, K., Zhang, M., Tang, F., Dong, W., Huang, F. and Xu, C. (2022) Transformers in computational visual media: A survey. Comput. Vis. Media, 8, 33-62, Bra 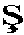 oveanu, A. M. and Andonie, R. (2020) Visualizing transformers for nlp: a brief survey. In. IEEE, pp. 270-279, and Khan, S., Naseer, M., Hayat, M., Zamir, S. W., Khan, F. S. and Shah, M. (2021) Transformers in vision: A survey. ACM Comput. Surv. CSUR*,* the contents of which are hereby incorporated by reference into the present disclosure). For the operation at this stage, a classical transformer model containing multi-head attention and positional encoding (see Vaswani, A., Shazeer, N., Parmar, N., Uszkoreit, J., Jones, L., Gomez, A. N., Kaiser, L. and Polosukhin, I. (2017) Attention is all you need. Adv. Neural Inf. Process. Syst., 30**)** can be directly used to train the protein subsequence-nucleic acid subsequence corpus (training set) prepared through stage 1, to realize direct end-to-end (or called sequence-to-sequence) translation from an amino acid sequence to a codon sequence. This step can also incorporate pre-trained models such as Bert and GPT/GPT-2/GPT-3 or use transformer variants (X-formers) (see Lin, T., Wang, Y., Liu, X. and Qiu, X. (2021) A survey of transformers. ArXiv Prepr. ArXiv210604554) for modeling. More generally, the neural machine translation model may be an artificial neural network machine translation model containing self-attention and positional encoding. The concepts of incorporation and pre-trained models are mentioned here. With regard to incorporation and pre-trained models, for example, encoders of the pre-trained protein model ProtBert in ProtTrans (see link: https://pubmed.ncbi.nlm.nih.gov/34232869/) and the pre-trained DNA model DNABert (see link: https://academic.oup.com/bioinformatics/article/3 7/15/ 2112/6128680) can be used to perform feature extraction on the input and output, respectively, which are then used as input and output embeddings, for example, see the introduction in the following link: https://arxiv.org/abs/2002.06823. The contents of these introductions and knowledge are hereby incorporated by reference into the present disclosure.

Next, continue to refer to FIG. 1, the method proceeds to steps S130, S140 and S150. In step S130, the protein sequence requiring codon optimization is cleaved into protein subsequences. After the translation tool (trained neural machine translation model) has been prepared through the previous stage, in step S140, the trained neural machine translation model is used to translate the protein subsequences from amino acid sequences to codon sequences. In step S150, the translated subsequences are overlapped, so as to combine same into a full-length codon sequence. During the overlapping of the subsequences to combine same into the codon sequence, the synonymous codon with the highest occurrence frequency or number is selected as the optimal codon for the position according to the frequency or the number of the synonymous codon corresponding to each amino acid position of the protein sequence. Specifically, see the detailed description of the translation process of the protein sequences below.

### Stage 3. Translation process of protein sequence

(1) Padding amino acid sequences are separately added at both ends of the protein sequence requiring codon optimization.

N padding amino acids are separately added at both ends of the protein sequence requiring codon optimization. The padding amino acids can consist of N amino acids randomly. As with the training process, the padding amino acids can be N consecutive M (methionine/met) or W (tryptophan). The reason for selecting M or W is that most of the highly expressed protein sequences contain M or W.

(2) A sliding window is used to cleave the protein sequence into protein subsequences.

During cleavage, generally, N = L1 - 1, i.e., the length of the sliding window is L1 amino acids and L1 = N + 1. The sliding step size is still 1 amino acid. The length L1 of the sliding window can be 5 to 22 amino acids.

FIG. 3 is a schematic diagram of an embodiment used during the implementation of the method of FIG. 1. When the length of the sliding window is 3 amino acids, the sliding step size is 1, the sequence is read sequentially, and each amino acid is read 3 times. That is, as illustrated in FIG. 3, N = 2 and L1 = 3 amino acids, as in the manner of training corpus processing, a protein sequence of length n is cleaved into protein subsequences by a sliding window, and by adding the padding amino acid sequences, each amino acid of the protein sequence can be scanned the same number of times by the sliding window, that is, each amino acid will be included in the L1 cleaved protein subsequences.

As with the training process, generally, the length of the sliding window is consistent with the main length of the ribosome-binding fragment in ribosome profiling. Correspondingly, the sliding step size of the sliding window is consistent with a protein biosynthesis process in which the ribosome slides along the mRNA by the length of a single codon during protein translation.

(3) The trained neural machine translation model is used to translate the protein subsequences from amino acid sequences to codon sequences.

Since each amino acid will be included in the L1 cleaved protein subsequences, the amino acid at each position of the protein sequence will be translated L1 times by the neural machine translation model trained in step S120.

(4) The translated subsequences are overlapped, so as to combine same into a full-length codon sequence.

Finally, after L1 translations of a certain amino acid, the synonymous codon with the highest occurrence frequency (or number) is selected as the optimal codon for the position. That is, the synonymous codon with the highest occurrence frequency or number is selected as the optimal codon for the position according to the frequency or the number of the synonymous codon corresponding to each amino acid position of the protein sequence.

That is, during the overlapping of the translated subsequences to combine same into the full-length codon sequence, the synonymous codon with the highest occurrence frequency or number after L1 translations of each amino acid in the L1 subsequences of the protein sequence is selected as the optimal codon for the amino acid position, and thus the sequence consisting of the optimal codons for all amino acids is used as the synthesized full-length codon sequence.

For a certain amino acid position, when two or more synonymous codons occur at the same frequency or number, selection may be made according to other screening criteria, such as codon preference, GC%, and folding energy ordering of nucleic acid sub-fragments. For example, the selection may depend on downstream applications: if designing an mRNA vaccine, codons with fewer T can be selected, or those forming a smaller or larger number of CG pairs with the upstream and downstream sequences, etc. can be considered.

In addition, according to a preferred embodiment of the present invention, the above method can continue to comprise the following preferred processing mode. That is, synonymous codon substitution is performed for deleterious feature sequences, enzyme cleavage sites for molecular cloning operations, or regions capable of producing alternative splicing sites in expression hosts.

### Stage 4. Filtration of deleterious sites and alternative splicing sites

For reported deleterious feature sequences (motifs), enzyme cleavage sites for molecular cloning operations, and regions capable of producing alternative splicing sites in expression hosts, potential risks are reduced or eliminated by performing synonymous codon substitutions for these regions in the optimized sequence.

### Beneficial technical effect

In addition to the benefits already mentioned above, those skilled in the art will recognize from the teachings and examples of the present disclosure that: the size of the sliding window is determined based on Ribo-Seq, the interaction between ribosomes and mRNA in the biological process of protein synthesis is simulated, subsequences are generated using the sliding window, the complexity of neural machine translation is reduced and the effectiveness is improved by continuous local optimization, and meanwhile, self-attention is used in the transformer or variants thereof, such that the translation effect can be improved. Finally, the optimal codon is selected according to the occurrence number/frequency of synonymous codons at a certain position, which essentially integrates the process of local folding and local unfolding of mRNA, and features such as the codon usage bias and dicodon usage bias involved in this process.

### Evaluation

Data show that the GenSmart codon optimization tool works better than other common tools. Moreover, the GenSmart codon optimization tool (see WO 2020/024917 A1) has performed well in multiple evaluations of codon optimization algorithms in different host expression systems and different application fields such as gene cell therapy. See, for example, the description of the following documents, the contents of which are hereby incorporated by reference into the present disclosure:
Goulet, D. R., Yan, Y., Agrawal, P., Waight, A. B., Mak, A. N. and Zhu, Y. (2022) Codon Optimization Using a Recurrent Neural Network. J. Comput. Biol.*;* Ranaghan, M. J., Li, J. J., Laprise, D. M. and Garvie, C. W. (2021) Assessing optimal: inequalities in codon optimization algorithms. BMC Biol., 19, 1-13
Gao, Z., Ravendran, S., Mikkelsen, N. S., Haldrup, J., Cai, H., Ding, X., Paludan, S. R., Thomsen, M. K., Mikkelsen, J. G. and Bak, R. O. (2022) A truncated reverse transcriptase enhances prime editing by split AAV vectors. Mol. Ther.
Dreismann, A. K., McClements, M. E., Barnard, A. R., Orhan, E., Hughes, J. P., Lachmann, P. J. and MacLaren, R. E. (2021) Functional expression of complement factor I following AAV-mediated gene delivery in the retina of mice and human cells. Gene Ther., 28, 265-276
Karaiyan, P., Chang, C. C. H., Chan, E.-S., Tey, B. T., Ramanan, R. N. and Ooi, C. W. (2022) In silico screening and heterologous expression of soluble dimethyl sulfide monooxygenases of microbial origin in Escherichia coli. Appl. Microbiol. Biotechnol., 106, 4523-4537

Therefore, in this section, *E.coli* BL-21 (DE3) is used as an expression system, the expression of protein M2-1 (NCBI Accession number: Q2Y2M2.1, with sequence set forth in SEQ ID NO: 1 and the C-terminus comprising a His tag sequence) is used as a test case, the sequence optimized using the GenSmart codon optimization tool is as set forth in SEQ ID NO: 2, and the sequence optimized using the method of the present invention is as set forth in SEQ ID NO: 3. Protein expression is induced by IPTG for 16 hours at 15°C after processing with the two methods, respectively, and the codon optimization effects of the GenSmart codon optimization tool and the codon optimization process of the present invention are compared. Sequence information is given below in relation to the sequences of SEQ ID NO: 1, SEQ ID NO: 2 and SEQ ID NO: 3, respectively.

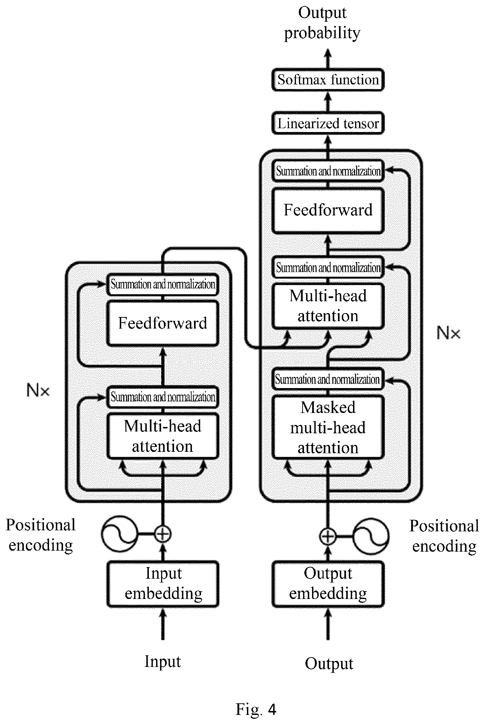

The structure of the transformer model is as shown in FIG. 4, which is consistent with the document. For the specific description of the structure, reference may also be made to the relevant document cited by the above transformer model, which will not be repeated here. In the above example, the size of the nucleic acid sliding window is 30 nt, the size of the protein sliding window is 10 amino acids (for protein sequences), the nucleic acid sliding window slides from the 5' end to the 3' end with a step size of 3 nt each time, and the protein sliding window moves one amino acid from the N-terminus (amino-terminus) to the C-terminus (carboxyl-terminus) each time; the left and right ends of the subsequence are: the protein subsequence is padded with 9 M and the nucleic acid subsequence is padded with 9 ATG. The multi-head modules of the self-attention in the transformer model all have 8 heads, both the coding layer and decoding layer consist of 6 layers, the size of each batch is 128 during training, the training epoch is 500, the length of the word embedding vector is 512, the drop ratio is 0.1, the size of the fully connected layer is 2048, the activation function is Relu, the eps for layer normalization is 0.00001, the tokens for the protein sequence are abbreviations of 20 amino acids, an asterisk (which may represent a stop code) and sentence start and end flags, and the tokens for the nucleic acid sequence are 64 codons and sentence start and end flags. The above are common hyperparameters in AI training models, which can have an impact on the final effect of the model. If these hyperparameters are not properly selected, the model architecture may be good but the use effect is not good. These parameters are hyperparameters obtained after learning based on the training set. In fact, different parameters can be obtained by inputting different training sets. These parameters are parameters that have shown good effects in the present application, but the present application is not limited to these parameters.

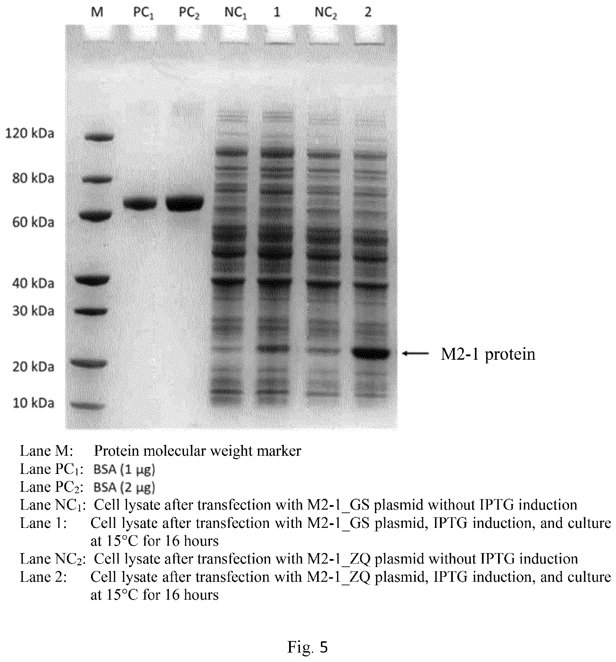

After expression, the whole bacterial samples are tested by SDS-PAGE. The results are as shown in FIG. 5, and the expression levels are as shown in the table below.

Consistent with the results of this example, the algorithm of the present invention has achieved an overall increase in protein expression level compared with the GenSmart codon optimization algorithm in more than 100 protein expression experiments.

| Plasmid name | Optimization tool | Lane | Expression vector | *E. coli* strain | Expression condition | Expression level |
|---|---|---|---|---|---|---|
| M2-1_GS | GenSmart codon optimization tool | Lane 1 | pET-30a (+) | BL21 (DE3) | 15°C, IPTG induction for 16 hours | 2 mg/L |
| M2-1_ZQ | The optimization algorithm of the present invention | Lane 2 | pET-30a (+) | BL21 (DE3) | 15°C, IPTG induction for 16 hours | 20 mg/L |

The protein sequences corresponding to the two plasmids are the same, and they only have different encoding sequences obtained by different optimization algorithms. Therefore, the increase in expression level is independent of the protein sequence itself. It is mentioned in the Background Art that the effect of RNN-based AI codon optimization is only comparable to that of GenSmart. According to the above evaluation results, the effect of the present invention has surpassed that of GenSmart, which indirectly indicates that the effect of the present invention is better than that of RNN-based AI codon optimization.

The specific experimental process for expression evaluation is as follows:

### 1) Transformation

BL21 (DE3) competent cells were taken from an ultra-low temperature freezer and thawed on ice. Plasmid (100 ng) was added to the competent cells and the resulting mixture was gently pipetted for uniform mixing. Cells in two tubes were transformed with the M2-1_GS plasmid, and cells in the other two tubes were transformed with the M2-1_ZQ plasmid. The cells were placed on ice for 30 minutes, then placed in a water bath kettle at 42°C for heat shock for 90 seconds, and then placed on ice for 3 minutes. Subsequently, 100 µl of LB liquid medium at room temperature was added. After oscillation culture in a shaker at 37°C and 200 rpm for 60 minutes, the bacterial solution was uniformly mixed and then plated on kanamycin-resistant plates. The plates were inverted and cultured at 37°C overnight.

### 2) Small-scale expression

Two single clones were separately picked and inoculated into test tubes to which 4 ml of LB containing 50 µg/ml kanamycin was added, and then oscillation culture was performed in a shaker at 37°C and 200 rpm. When the OD600 reached 0.6 to 0.8, 0.5 mM IPTG was added into each of two test tubes (the cells in one tube were transformed with the M2-1_GS plasmid, and the cells in the other tube were transformed with the M2-1_ZQ plasmid) (the expression results correspond to lanes 1 and 2 in FIG. 4, respectively), and culture was performed at 15°C for 16 hours. The cells in the other two test tubes (the cells in one tube were transformed with the M2-1_GS plasmid, and the cells in the other tube were transformed with the M2-1_ZQ plasmid) were not subjected to IPTG induction, as negative controls (the expression results correspond to lanes NC1 and NC2 in FIG. 4, respectively).

### 3) Sample preparation and SDS

The pellet resulting from centrifugation of 450 µl of medium was taken, resuspended in 300 µl of lysis buffer (50 mM Tris-HCl, 150 mM NaCl, 5% glycerol, pH 8.0), and sonicated for 1 minute. 100 µl of lysate was taken and mixed uniformly with 50 µl of 5×loading buffer. The resulting mixture was heated at 100°C for 10 minutes and centrifuged at 15000 rpm for 5 minutes. Then, protein expression was detected by SDS-PAGE (see FIG. 5).

In addition, those of ordinary skill in the art will recognize that the method of the present disclosure may be implemented as a computer program. As described above with reference to the accompanying drawings, the method of the above example is performed by one or more programs in which instructions cause a computer or processor to execute the algorithm described with reference to the accompanying drawings. These programs may be stored using various types of non-transitory computer-readable media and provided to a computer or processor. Non-transitory computer-readable media include various types of tangible storage media. Examples of non-transitory computer-readable media include magnetic recording media (such as floppy disks, magnetic tapes, and hard disk drives), magneto-optical recording media (such as magneto-optical disks), CD-ROM (compact disk read-only memory), CD-R, CD-R/W and semiconductor memories (such as ROM, PROM (programmable ROM), EPROM (erasable PROM), flash ROM and RAM (random access memory). Further, these programs may be provided to a computer by using various types of transitory computer-readable media. Examples of transitory computer-readable media include electrical signals, optical signals, and electromagnetic waves. Transitory computer-readable media may be used to provide programs to a computer via wired communication paths (such as electric wires and optical fibers) or wireless communication paths.

For example, according to an example of the present disclosure, a non-transitory computer-readable storage medium for storing a computer program may be provided, wherein the computer program comprises instructions that, when executed by a processor of an electronic device, cause the electronic device to implement the method as described above.

Additionally, according to the content disclosed in the present invention, a system for optimizing a nucleic acid sequence for expression of a protein in a host may also be proposed, the system comprising: a processor; a memory; and a computer program. The computer program is stored in the memory and configured to be executed by the processor. The computer program comprises instructions for implementing the method described above.

In addition, the present invention also relates to an electronic device for optimizing a nucleic acid sequence for expression of a protein in a host, wherein the device comprises a tool for implementing the method as described above.

A computer program product stored on a recordable medium for optimizing a nucleic acid sequence for expression of a protein in a host may also be proposed, the computer program product comprising computer software for implementing the method as described above.

In another aspect, according to the content disclosed above, the present invention may also have the following applications. For example, an isolated nucleic acid molecule comprising the optimized nucleic acid sequence obtained by the method as described above may be proposed.

A vector comprising the isolated nucleic acid molecule as described above may also be proposed.

Additionally, a recombinant host cell comprising the isolated nucleic acid molecule as described above or the vector as described above is proposed.

In addition, according to an example of the present invention, a method for expressing a protein in a host cell is proposed, the method comprising: (a) using the method as described above to obtain an optimized nucleic acid sequence for expression of the protein in the host cell; (b) synthesizing a nucleic acid molecule comprising the optimized nucleic acid sequence; (c) introducing the nucleic acid molecule into the host cell to obtain a recombinant host cell; and (d) culturing the recombinant host cell under conditions that allow expression of the protein from the optimized nucleic acid sequence.

The embodiments of the present invention are not limited to the above examples, and various modifications and improvements in forms and details may be made to the present invention by those of ordinary skill in the art without departing from the spirit and scope of the present invention, and these modifications and improvements are considered to fall within the scope of protection of the present invention.

The sequence information is as follows:
SEQ ID NO: 1 M2-1 protein 
MSRKAPCKYEVRGKCNRGSECKFNHNYWSWPDRYLLLRSNYLLNQL
LRNTDRSDGLSLISGAGRDDRTQDFVLGSTNVVQNYIDNNENITKASACYS
LYNIIKQLQETDVRQARDNKVDDSKHVALHNLVLSYMEMSKTPASLINNL
KKPPKEKLKKLAKLIIELSAGVENDSTAAMQDSANSDHHHHHH
SEQ ID NO: 2 Sequence optimized by GenSmart 
ATGTCAAGGAAGGCTCCCTGTAAATATGAAGTGCGCGGCAAGTGC
AACAGAGGTAGCGAGTGCAAGTTTAATCATAATTACTGGTCTTGGCCA
GATCGTTATCTGTTGCTACGCAGCAACTACTTGTTGAATCAGTTGCTGC
GTAATACCGACCGTAGCGACGGCCTGTCGCTGATCAGCGGTGCAGGTC
GTGACGACCGCACCCAGGACTTCGTGCTGGGTTCGACTAACGTGGTCC
AGAATTACATTGATAACAACGAAAATATCACCAAAGCGTCTGCGTGTT
ATAGCTTATATAACATTATTAAACAGCTGCAAGAGACCGATGTTCGTCA
AGCGCGTGACAACAAAGTTGATGACTCCAAGCATGTTGCCCTGCATAA
CCTGGTTCTGTCCTACATGGAAATGAGCAAGACCCCGGCATCCCTGATT
AACAACCTGAAGAAACCGCCGAAAGAAAAACTCAAGAAGCTGGCGAA
ACTGATCATCGAGCTGAGCGCGGGCGTGGAGAACGATTCTACGGCTGC
CATGCAAGACAGCGCTAATAGCGATCACCACCACCACCACCATTAA
SEQ ID NO: 3 Sequence optimized by the method of the present invention

ATGAGCCGTAAAGCACCGTGTAAATATGAAGTTCGTGGTAAATGT
AATCGTGGTAGCGAATGTAAATTTAACCATAATTATTGGAGCTGGCCTG
ATCGTTATCTGCTGCTGCGTAGCAATTATCTGCTGAATCAGCTGCTGCG
TAATACCGATCGTTCAGATGGTCTGAGCCTGATTAGCGGTGCAGGTCGT
GATGATCGTACCCAGGATTTTGTTCTGGGTAGCACCAATGTTGTTCAGA
ACTATATCGATAACAACGAAAACATTACCAAAGCAAGCGCATGTTATA
GCCTGTATAACATTATTAAACAGCTGCAAGAAACCGATGTTCGTCAGG
CACGTGATAATAAAGTTGATGATAGCAAACATGTTGCCCTGCATAATCT
GGTTCTGAGCTATATGGAAATGAGCAAAACACCGGCAAGCCTGATTAA
TAACCTGAAAAAACCGCCTAAAGAAAAACTGAAAAAACTGGCAAAAC
TGATTATTGAACTGAGCGCAGGTGTTGAAAATGATAGCACCGCAGCAA
TGCAGGATAGCGCAAATAGCGATCATCATCATCATCATCATTAA

## Claims

1. A computer-implemented method for optimizing a nucleic acid sequence for expression of a protein in a host, **characterized in that** the method comprises:
obtaining a protein subsequence-nucleic acid subsequence pair according to collected highly expressed protein sequences and encoding nucleic acid sequences thereof, so as to form a training set;
using the training set to train a neural machine translation model, wherein the neural machine translation model is used to realize translation from an amino acid sequence to a codon sequence;
cleaving the protein sequence requiring codon optimization into protein subsequences;
using the trained neural machine translation model to translate the protein subsequences from amino acid sequences to codon sequences;
overlapping the translated subsequences, so as to combine same into a full-length codon sequence, wherein the synonymous codon with the highest occurrence frequency or number is selected as the optimal codon for the position according to the frequency or the number of the synonymous codon corresponding to each amino acid position of the protein sequence.

2. The method according to claim 1, **characterized in that** the step of forming a training set further comprises:
separately adding padding amino acid sequences at both ends of the protein sequence;
separately adding corresponding padding codons at both ends of the encoding nucleic acid sequence;
using a sliding window to perform subsequence extraction on the protein sequences and the encoding nucleic acid sequences thereof;
tokenizing the protein subsequences with amino acids as the minimum unit;
tokenizing the nucleic acid subsequences with codons as the minimum unit;
adding a sentence start flag and a sentence end flag before and after each of the protein subsequences and the nucleic acid subsequences, respectively, to form a training set.

3. The method according to claim 2, **characterized in that**
the padding amino acid sequence consists of N amino acids;
the length of the sliding window on the protein sequence is L1 amino acids and L1 = N + 1, with a sliding step size of 1 amino acid;
the length of the sliding window on the encoding nucleic acid sequence is L2 nucleotides and L2 = 3 × L1, with a sliding step size of 3 nucleotides.

4. The method according to claim 3, **characterized in that** the padding amino acid sequence consists of N consecutive methionine or N consecutive tryptophan.

5. The method according to claim 4, **characterized in that** when the padding amino acid is methionine, the corresponding padding codon is ATG; when the padding amino acid is tryptophan, the corresponding padding codon is TGG.

6. The method according to any one of claims 3-5, **characterized in that** the length L1 of the sliding window on the protein sequence is 5 to 22 amino acids, and the length L2 of the sliding window on the encoding nucleic acid sequence is 15 to 66 nucleotides.

7. The method according to any one of claims 1-6, **characterized in that** the neural machine translation model is a transformer model.

8. The method according to any one of claims 1-7, **characterized in that** the neural machine translation model is an artificial neural network machine translation model containing self-attention and positional encoding.

9. The method according to any one of claims 1-8, **characterized in that** the step of cleaving the protein sequence requiring codon optimization into protein subsequences further comprises:
separately adding padding amino acid sequences at both ends of the protein sequence requiring codon optimization;
using a sliding window to cleave the protein sequence into protein subsequences.

10. The method according to claim 9, **characterized in that**
the padding amino acid sequence consists of N amino acids;
the length of the sliding window is L1 amino acids and L1 = N + 1, with a sliding step size of 1 amino acid;
by adding the padding amino acid sequences, each amino acid of the protein sequence is scanned the same number of times by the sliding window, and each amino acid will be included in the L1 cleaved protein subsequences;
in the step of translating the protein subsequences from amino acid sequences to codon sequences, the amino acid at each position of the protein sequence is translated L1 times by the trained neural machine translation model.

11. The method according to claim 10, **characterized in that** the padding amino acid sequence consists of N consecutive methionine or N consecutive tryptophan.

12. The method according to claim 10 or 11, **characterized in that** the length L1 of the sliding window is 5 to 22 amino acids.

13. The method according to any one of claims 10-12, **characterized in that** in the step of overlapping the translated subsequences so as to combine same into a full-length codon sequence, the synonymous codon with the highest occurrence frequency or number after L1 translations of each amino acid in the L1 subsequences of the protein sequence is selected as the optimal codon for the amino acid position, and thus the sequence consisting of the optimal codons for all amino acids is used as the synthesized full-length codon sequence.

14. The method according to any one of claims 1-13, **characterized in that** for one amino acid position, when two or more synonymous codons occur at the same frequency or number, the optimal codon for the position is selected according to at least one of the following criteria: codon preference, GC%, and folding energy ordering of nucleic acid sub-fragments.

15. The method according to any one of claims 1-14, **characterized in that** the method further comprises:
performing synonymous codon substitution for deleterious feature sequences, enzyme cleavage sites for molecular cloning operations, or region sequences capable of producing alternative splicing sites in expression hosts.

16. A non-transitory computer-readable storage medium for storing a computer program, **characterized in that** the computer program comprises instructions that, when executed by a processor of an electronic device, cause the electronic device to implement the method according to any one of claims 1-15.

17. A system for optimizing a nucleic acid sequence for expression of a protein in a host, **characterized in that** the system comprises:
a processor;
a memory; and
a computer program, wherein the computer program is stored in the memory and configured to be executed by the processor, and the computer program comprises instructions for implementing the method according to any one of claims 1-15.

18. An electronic device for optimizing a nucleic acid sequence for expression of a protein in a host, **characterized in that** the device comprises a tool for implementing the method according to any one of claims 1-15.

19. A computer program product stored on a recordable medium for optimizing a nucleic acid sequence for expression of a protein in a host, **characterized in that** the computer program product comprises computer software for implementing the method according to any one of claims 1-15.

20. An isolated nucleic acid molecule, **characterized by** comprising the optimized nucleic acid sequence obtained by the method according to any one of claims 1-15.

21. A vector, **characterized by** comprising the isolated nucleic acid molecule according to claim 20.

22. A recombinant host cell, **characterized by** comprising the isolated nucleic acid molecule according to claim 20 or the vector according to claim 21.

23. A method for expressing a protein in a host cell, **characterized in that** the method comprises:
(a) using the method according to any one of claims 1-15 to obtain an optimized nucleic acid sequence for expression of the protein in the host cell;
(b) synthesizing a nucleic acid molecule comprising the optimized nucleic acid sequence;
(c) introducing the nucleic acid molecule into the host cell to obtain a recombinant host cell; and
(d) culturing the recombinant host cell under conditions that allow expression of the protein from the optimized nucleic acid sequence.
